# EUROPEAN PATENT APPLICATION

(11) **EP 0 888 785 A1**
(43) Date of publication of application: **07.01.1999**
(21) Application number: 98305093.1
(22) Date of filing: 29.06.1998
(51) Int. Cl.: A61L 15/46, A61L 15/24

(54) **Odor reducing wound dressings**

(30) Priority: 30.06.1997 GB 9713839
(71) Applicant: Johnson & Johnson Medical Ltd., Edinburgh EH2 4NH (GB)
(72) Inventor: Watt, Paul William, Steeton, West Yorkshire BD20 6UN (GB); Josephsen, Lotte, Riverside, Stirling FK8 1QS (GB); Grady, Michael William, Burley-in-Wharfedale LS29 7LP (GB); McGregor, James, Bishopbriggs, Glasgow G64 1AY (GB)
(74) Representative: Fisher, Adrian John

(57) **Abstract**

The invention provides odor reducing wound dressings comprising porous adsorbent polymer particles having an internal surface area of at least 500 m²/g, preferably at least 750 m²/g. The polymer particles are preferably porous, cross-linked polystyrene beads.

## Description

The present invention relates to improved odor reducing wound dressings.

Odor dressings for use in wound management of malodorous wounds, such as leg ulcers, fungating carcinomas, faecal fistulae and pressure sores, have traditionally used activated charcoal to adsorb the gaseous materials responsible for the undesirable odors. The charcoal is normally provided as a charcoal cloth inside a non-adherent, spun-bonded nylon sleeve, for example the product sold under the Registered Trade Mark ACTISORB by Johnson & Johnson. Such dressings can provide satisfactory odor control, but the black appearance of the dressing does not have a good psychological effect on the patients and nurses.

EP-A-0509409 describes absorbent pads for retaining human exudate that include odor-absorbing particles. The particles comprise an anhydrous, non-buffered blend of at least basic and pH-neutral odor absorbing particles. The basic odor control particles absorb acidic odor molecules, and preferably include inorganic salts of carbonates, bicarbonates, phosphate, biphosphate, sulfate, bisulfate and mixtures thereof. The pH-neutral odor adsorbing materials include-activated carbon, clays, silicas, diatomaceous earth, polystyrene derivatives, zeolites, molecular sieves and starches. The preferred materials are sodium bicarbonate and synthetic zeolites, respectively. A preferred zeolite is commercially available from UOP under the Registered Trade Mark ABSCENTS. In addition, the absorbent article can contain acidic particles to absorb malodorous alkaline vapours, such as the amines putrescine and cadaverine. Preferred acidic materials include ascorbic acid, stearic acid, boric acid and, especially, citric acid.

GB-A-2259858 describes an odor absorbing sachet containing a natural zeolite material for absorbing odours from malodorous wounds.

It has now been found that wound dressings containing polymer particles having a very high internal surface area can provide an inexpensive, highly efficacious and visually attractive means for the treatment of malodorous wounds.

Accordingly, the present invention provides an odor reducing wound dressing comprising adsorbent porous polymer particles having a surface area of at least 500m²/g.

Here and elsewhere in the specification, the surface area of the polymer particles refers to the surface area as determined by BET single point (dry sample) nitrogen adsorption measurements.

Here and elsewhere in the specification the term "wound dressing" encompasses, in addition to conventional wound dressing formats, all absorbent articles for the treatment of human exudate, including articles for absorbing menstrual fluid, urine, and wound exudate, as well as absorbent articles for use by colostomy patients.

Preferably, the polymer particles have a surface area of at last 750m²/g, more preferably at least 1000m²/g.

Preferably, the polymer particles have a median pore diameter, as determined by mercury porosimetry, of less than 1.0µm, more preferably less than 0.25µm, and most preferably less than 0.1µm.

The polymer particles may be in the form of a powder having particle size 10-150µm. Preferably, the polymer particles comprise beads having diameters in the range 0.1-5.0mm, preferably 1.0-4.0mm. Such beads may be commercially available for use as molecular sieves or as the stationary phase for HPLC.

Preferably, the polymer particles comprise polystyrene, and more preferably the polymer particles consist essentially of cross-linked polystyrene. Suitable, highly porous cross-linked polystyrene particles are available under the Registered Trade Marks PUROSEP and MACRONETS from, Purolite International Limited, Cowbridge Road, Pontyclun, Mid-Glamorgan, U.K. The Synthesis of these materials is described, for example, in SU-A-948110, SU-A-434757 and SU-A-804647. These materials have been used in the chemical engineering art as molecular sieves for the removal of impurities from liquids, but they have not hitherto been suggested for use in wound dressings.

The porous cross-linked polystyrene particles are formed by treatment of polystyrene, or its copolymers with 0.3-2% of divinylbenzene, with a bifunctional cross-linking agent in the presence of Friedel-Crafts catalysts in an organic solvent. The resulting cross-linked gel is washed and dried to provide the absorbent particles. Preferred bifunctional cross-linking agents include p-xylylene dichloride, monochlorodimethyl ether and dimethylformal. Preferred Friedel-Craft catalysts include iron trichloride, aluminium trichloride, tin tetrachloride and zinc dichloride. Preferred organic solvents include dichloroethane, tetrachloroethane and nitrobenzene.

Preferably, the odor reducing wound dressing according to the present invention comprises the adsorbent polymer particles as specified above enclosed in a container formed wholly or in part from a woven, nonwoven or knitted fabric. More preferably, the particles are enclosed in an envelope, sleeve or sock formed entirely from the said fabric. Preferred fabrics are spun-bonded, non-woven, non-adherent nylon fabrics of the kind already used for wound contacting purposes. The edges of the cloth are preferably held together by heat sealing.

In alternative embodiments, the adsorbent porous polymer particles may be coated or otherwise combined with an antiseptic agent, or may be directly incorporated in an antiseptic paste, such as chlorhexidine paste. Alternatively, the particles may be incorporated in a hydrophilic absorbent foam for use in wound dressings, such as the polyurethane foam available from Johnson & Johnson under the Registered Trade Mark TIELLE. In yet other embodiments, the porous polymer particles may be dispersed in a biopolymer film or sponge, for use as or in a wound dressing.

A particular advantage of the odor reducing wound dressings according to the present invention is that they do not require a mixture of acidic, basic and neutral odor absorbent materials to achieve satisfactory results. The use of very high surface area adsorbent porous polymer particles as adsorbents achieves control of all acidic, basic and neutral odor-causing molecules. The polymer itself may additionally be functionalized so that it chemisorbs specific odor causing molecules, such as acidic or basic molecules.

Specific embodiments of the present invention will now be described further, by way of example, with reference to the accompanying drawings, in which:-
Figure 1 shows a graph of vapour phase concentration of diethylamine above dry odor reducing dressings versus time for: a dressing according to the present invention (solid squares), a dressing containing activated carbon (solid triangles) and a control experiment with no odor adsorbent (open squares);
Figure 2 shows a graph similar to that in figure 1, but for wet odor reducing dressings instead of the dry dressings in the graph of Figure 1; and
Figure 3 shows a graph similar to that if Figure 2, for a wet activated carbon dressing (solid triangles) and a wet dressing according to the present invention (solid squares).

### Procedure 1

A cross-linked polystyrene molecular sieve having very high internal surface area and uniform pore size is prepared as follows:

A solution of 2.6 g (0.01 mole) of SnCl₄ in 100 ml of tetrachloroethane is added to a solution of 104 g (1 mole) of polystyrene and 0.76 g (0.01 mole) of dimethylformal in 700 ml of the same solvent. The mixture is thoroughly stirred and held for 5 hours at room temperature. The resultant gel is broken up, washed with acetone, then with a mixture of acetone and 0.5N HCl, then with 0.5N HCl, then with water, and the gel is then dried.

### Example 1

An odor reducing wound dressing according to the present invention is prepared as follows.

Approximately 1.5 g of PUROSEP beads supplied by Purolite International, are placed between two sheets of spun-bonded, non-woven nylon web, and the edges of the nylon web are heat bonded together to provide a flat envelope (i.e. sachet, or "tea-bag") containing the PUROSEP beads enclosed therein. This dressing can then be applied directly to a malodorous wound, as or in part of a wound dressing optionally comprising other absorbent and/or protective wound dressing layers.

### Example 2

The superiority of the porous, cross-linked polystyrene PUROSEP beads as odor adsorbents is demonstrated as follows.

Canisters are packed with cotton wool. 150ml of 0.3% v/v diethylamine solution is introduced into each canister so that the cotton wool is saturated. Upper canisters are prepared that are provided with a barrier layer extending across their bottom of a wound dressing prepared as in Example 1. Control upper canisters are prepared with no barrier layer. Comparative upper canisters are prepared with a barrier layer of ACTISORB (Registered Trade Mark of Johnson & Johnson) activated charcoal odor adsorbent wound dressings. One of the upper canisters is bolted to each assembled lower canister, and a 250 microliter vapour sample is taken immediately from the upper canister via an injection port (time zero). The canisters are transferred, bolted together, to an incubator at 37°C. Samples are subsequently taken at 1, 2, 3, 4, 5 and 6 hours from the same injection port. Each sample is analysed for diethylamine vapour using gas chromatography.

As the excess of diethylamine in each first canister diffuses into each second canister, it is initially adsorbed by the odor adsorbent compounds. However, as the amount of diethylamine in the upper canister increases, so does the partial pressure of diethylamine vapour in the upper canister, and it is this increase in partial pressure that is detected at intervals over 6 hours.

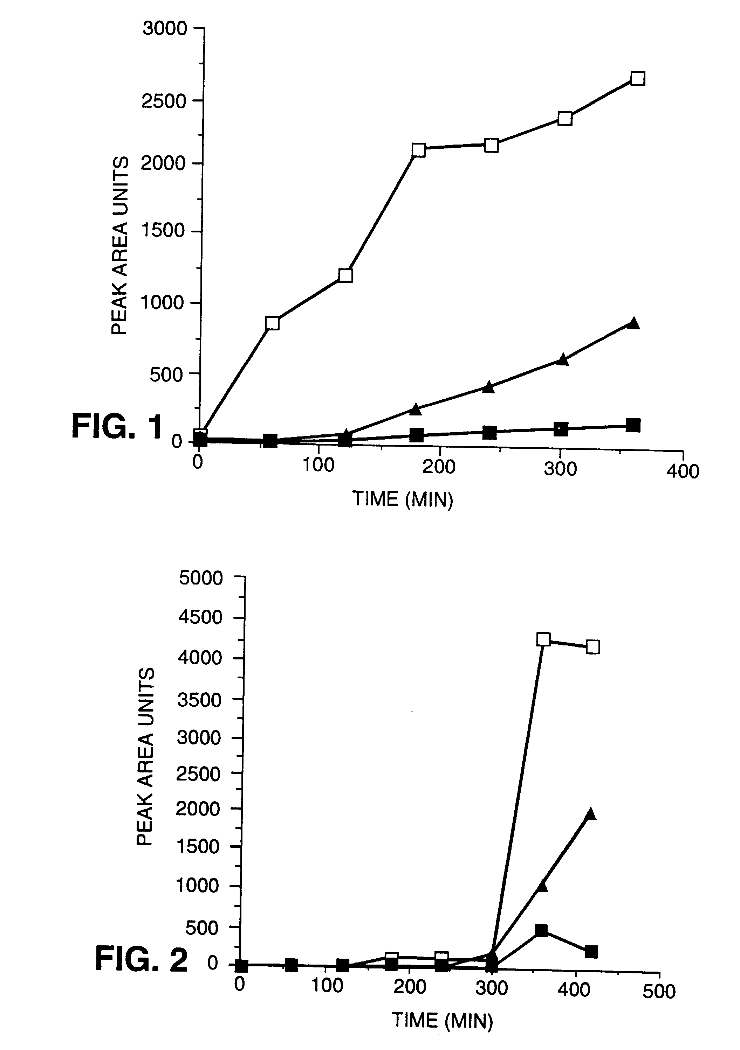

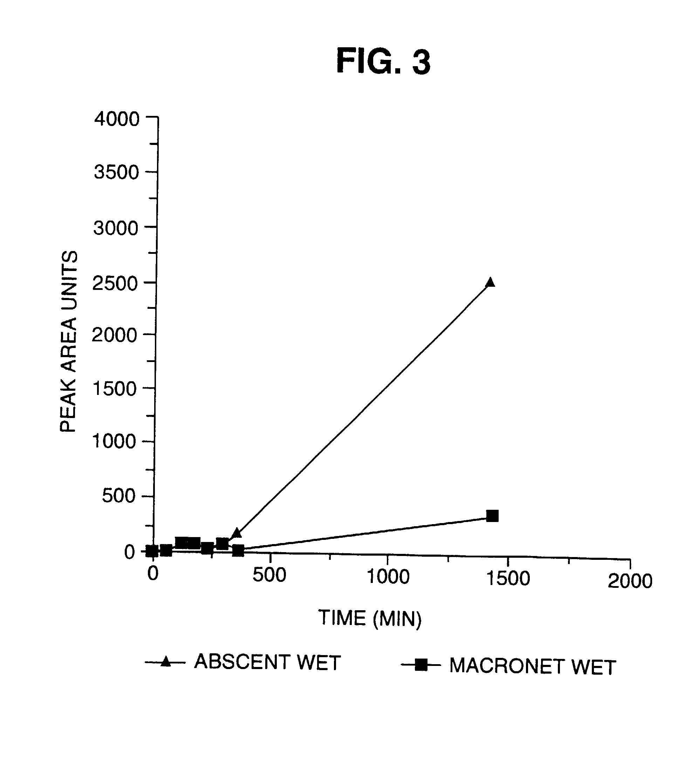

The results are shown in Figures 1 to 3. These drawings plot the GC peak area for diethylamine from the upper canister sample, plotted against time.

It can be seen that the vapour pressure of free diethylamine increases much more slowly over the wound dressings according to the present invention (solid squares) that it does over the activated charcoal odor absorbent dressing (solid triangles) currently used in the art. The control sample (no adsorbent) is shown in open squares.

The above embodiment has been described by way of example only. Many other embodiments falling within the scope of the accompanying claims will be apparent to the skilled reader.

## Claims

1. An odor reducing wound dressing comprising porous adsorbent, polymer particles having an internal surface area of at least 500m²/g.

2. An odor reducing wound dressing according to claim 1, wherein the polymer particles have an internal surface area of at least 750m²/g.

3. An odor reducing wound dressing according to claim 1 or 2, wherein the polymer particles have a median pore diameter, as determined by mercury porosimetry, of less than 1.0µm.

4. An odor reducing wound dressing according to any preceding claim, wherein the polymer particles comprise beads having diameters in the range 0.1-5.0mm.

5. An odor reducing wound dressing according to any preceding claim, wherein the polymer particles comprise polystyrene.

6. An odor reducing wound dressing according to claim 5, wherein the polymer particles consist essentially of cross-linked polystyrene.

7. An odor reducing wound dressing according to claim 6, wherein the porous, crosslinked polystyrene has a specific gravity of 1.0-1.1g/ml.

8. An odor reducing wound dressing according to any preceding claim, wherein the polymer particles are enclosed in a gas-permeable structure formed wholly or in part from a woven, nonwoven or knitted fabric.

9. An odor reducing wound dressing according to any of claims 1 to 7, wherein the polymer particles are dispersed in a gel or foam matrix.

10. An odor reducing wound dressing according to any preceding claim, wherein said polymer particles are pH neutral polymer particles.

11. An odor reducing wound dressing according to any preceding claim which is sterile and packaged in a bacteriaimpermeable package.

12. Use of porous adsorbent polymer particles having an internal surface area of at least 500m²/g for the preparation of a dressing for the treatment of malodorous wounds.
